# EUROPÄISCHE PATENTSCHRIFT

(11) **EP 1 309 353 B1**
(45) Veröffentlichungstag und Bekanntmachung des Hinweises auf die Patenterteilung: **05.10.2005**
(21) Anmeldenummer: 01960130.1
(22) Anmeldetag: 24.07.2001
(51) Int. Cl.: A61K 47/10, A61K 47/26, A61K 35/78

(54) **FLAVONHALTIGE PFLANZENEXTRAKTLÖSUNGEN MIT VERBESSERTER LAGER- UND HITZESTABILITÄT**
FLAVONE-CONTAINING PLANT EXTRACT SOLUTIONS HAVING IMPROVED STORAGE AND HEAT STABILITY
SOLUTIONS D'EXTRAITS DE PLANTES CONTENANT DES FLAVONES PRESENTANT UNE STABILITE AU STOCKAGE ET A LA CHALEUR AMELIOREE

(30) Priorität: 16.08.2000 DE 10040610
(43) Veröffentlichungstag der Anmeldung: 14.05.2003
(73) Patentinhaber: Dr. Willmar Schwabe GmbH & Co., 76227 Karlsruhe (DE)
(72) Erfinder: HERRMANN, Joachim, 68789 St. Leon-Rot (DE); OSCHMANN, Rainer, 76829 Landau (DE); STUMPF, Heinz, 76228 Karlsruhe (DE); THÖLE, Marc, 76135 Karlsruhe (DE)
(74) Vertreter: Rudolph, Ulrike
(86) Internationale Anmeldenummer: PCT/DE2001/002871
(87) Internationale Veröffentlichungsnummer: WO 2002/013869

(56) Entgegenhaltungen:
- EP-A- 0 477 968
- DE-A- 2 117 429
- DE-B- 1 182 776
- GB-A- 820 788
- DEMROW HS ET AL: "Administration of wine and grape juice inhibits in vivo platelet activity and thrombosis in stenosed canine coronary arteries." CIRCULATION, Bd. 91, Nr. 4, 1995, Seiten 1182-1188, XP002192947 ISSN: 0009-7322

## Beschreibung

Die Erfindung betrifft sterile, injizierbare flavonhaltige Pflanzenextrakt-Lösungen mit hoher Lager- und Hitzestabilität bezüglich ihres Gehalts an pharmazeutisch relevanten Inhaltsstoffen (insbesondere an Flavonen und/oder Terpenen), diese enthaltende injizierbare Arzneimittel und Verfahren zu deren Herstellung.

Flavonhaltige Extrakte aus Pflanzenteilen werden in Form von Injektionslösungen zur Behandlung verschiedener Erkrankungen eingesetzt, zum Beispiel bei Hirnleistungsstörungen (Ginkgo-Extrakt), Venenleiden (Aesculus-Extrakt) und nachlassender Leistungsfähigkeit des Herzens (Crataegus-Extrakt).

Bei diesen injizierbaren Extraktlösungen besteht jedoch zum einen das Problem der unbefriedigenden Lagerstabilität sowohl aufgrund chemischer Instabilitäten, nämlich einer Abnahme der Konzentration der wirksamkeitsrelevanter Inhaltsstoffe, als auch aufgrund physikalischer Instabilitäten, nämlich Ausfällungen, Ausflockungen u. ä.. Ein weiteres Problem besteht darin, daß diese injizierbaren Extraktlösungen am einfachsten und zuverlässigsten mittels Hitzebehandlung sterilisiert werden, wobei jedoch gerade hier die Gefahr des Abbaus der relevanten Inhaltsstoffe besteht. Andere Herstellungsverfahren sind die aseptische Herstellung der Lösungen mit anschließender Entkeimungsfiltration und eventuell noch nachfolgender Lyophilisation. Diese Verfahren sind aber technisch aufwendig und vergleichsweise teuer.

DE-2117429-A offenbart Ginkgo-Extrakt enthaltende Injektionslösungen.

Aufgabe der vorliegenden Erfindung ist die Bereitstellung injizierbarer flavonhaltiger Pflanzenextraktlösungen (Injektionszubereitungen flavonhaltiger Pflanzenextrakte), die eine verbesserte Lager- und Hitzestabilität aufweisen, und die Bereitstellung von injizierbaren Arzneimitteln, welche solche flavonhaltigen Pflanzenextraktlösungen enthalten und selbst eine verbesserte Lagerungsstabilität aufweisen.

Eine Lösung dieser Aufgabe besteht in der Bereitstellung einer injizierbaren flavonhaltigen Pflanzenextrakt-Lösung aus Gingko biloba und/oder Aesculus spec., vorzugsweise aus deren Blätter und/oder Blüten,
dadurch gekennzeichnet,
- daß die flavonhaltigen Pflanzenextrakte in einem wäßrig-alkoholischen Lösungsmittel mit einem Gehalt von 2 - 40 % m/m, bevorzugt 10 - 25 % m/m Alkohol in Wasser gelöst sind (vorliegen),
- daß der pH-Wert der Lösung im sauren Bereich von pH 2 bis pH 6, vorzugsweise von pH 3 bis pH 4 liegt,
- daß die Lösung dampfsterilisiert ist, wobei nach 15 Minuten Dampfsterilisation bei 121°C und 2 bar der Gehalt an Flavonen und Terpenen um maximal 10%m/m verringert ist.

Als Alkohol eignet sich insbesondere Ethanol und/oder Propylenglykol.

Die Einstellung des pH-Wertes der Pflanzenextraktlösung erfolgt vorzugsweise durch Zugabe einer pharmazeutisch akzeptablen d.h. in Arzneimitteln üblicherweise verwendeten Säure, insbesondere Salzsäure. Anstelle oder neben Salzsäure kommen auch Phosphorsäure und/oder Essigsäure in Betracht.

Die erfindungsgemäßen Pflanzenextraktlösungen sind überraschenderweise sehr stabil gegenüber Hitzebehandlung, insbesondere Hitze- bzw. Dampfsterilisation (Autoklavierung), und gegenüber Langzeitlagerung. Ihr Gehalt an pharmazeutisch relevanten Inhaltsstoffe, insbesondere an Flavonen und/oder Terpenen, ist nach Dampfsterilisation, insbesondere unter Standardbedingungen bei 121°C, 2 bar für die Dauer von 15 Minuten, gegenüber dem Gehalt vor der Dampfsterilisation um maximal 10 % m/m verringert. Im Fall von Ginkgo-Extraktlösungen führt eine solche Dampf- bzw. Hitzesterilisation nachweislich weder bei den Flavonglykosiden noch bei den Terpenlactonen (Bilobalid, Ginkgolide) zu einer wesentlichen Beeinträchtigung ihrer Konzentrationen und Konstitutionen (vgl. Beispiel 1).

Werden derart dampf- bzw. hitzesterilisierte Pflanzenextraktlösungen in Behältnissen (beispielsweise Ampullen oder Kammern eines Mehrkammersystems) gelagert, ist ihr Gehalt an pharmazeutisch relevanten Inhaltsstoffen selbst nach 12 Monaten Lagerung unter Standardbedingungen (25°C Raumtemperatur und 60% relative Luftfeuchtigkeit) gegenüber dem Gehalt vor bzw. zu Beginn der Lagerung um maximal 10 % m/m verringert.

Zur weiteren Lösung der genannten Aufgabe wird ein Arzneimittel vorgeschlagen, das flavonhaltige Extraktlösungen aus Pflanzenteilen enthält, injizierbar ist (d.h. als Injektion verabreicht werden kann) und eine hohe Lagerungs- und Hitzestabilität bezüglich seines Gehalts an pharmazeutisch relevanten Wirkstoffen aufweist, und das sich dadurch auszeichnet, daß es ganz oder nahezu ausschließlich aus den beiden Komponenten (A), nämlich einer Pflanzenextrakt-Lösung nach einem der Ansprüche 1 bis 4 und (B), nämlich einer sterilen Puffersalzlösung (d.h. einer Pufferlösung, bestehend aus einem oder mehreren Puffersalzen) mit physiologisch-verträglicher, d.h. in Arzneimitteln üblicherweise verwendeten Stoffzusammensetzung besteht, wobei diese Komponenten bei (im Zeitpunkt) der Applikation miteinander vermischt sind, und wobei diese beiden Komponenten (A) und (B) separat hergestellt und bis zur Applikation, d.h. bis zum Zeitpunkt der beabsichtigten Applikation, voneinander getrennt in separaten Behältnissen (beispielsweise in zwei Ampullen oder in zwei Kammern einer Mehrkammerspritze oder eines anderen Mehrkammersystems) gelagert werden, und wobei der Gehalt an Flavonen und Terpenen der Komponente A nach 12 Monaten Lagerung bei 25°C und 60% Luftfeuchtigkeit um maximal 10% m/m verringert ist.

Als Pufferlösung (mit geeignetem pH-Wert und geeigneter Osmolarität) kommt bevorzugt eine Phosphorsäure/Natriumphosphat-Lösung in Betracht, die zudem Isotonisierungsmittel wie Kochsalz oder Zuckeralkohole enthalten kann.

Zur weitergehenden Erhöhung der Hitze- und Lagerstabilität des erfindungsgemäßen Arzneimittels können die Komponente(n) (A) und/oder (B) zudem Ascorbinsäure und/oder wenigstens einen anderen pharmazeutisch akzeptablen, d.h. in Arzneimitteln üblicherweise verwendeten Hilfsstoff enthalten.

Das erfindungsgemäße direkt applizierbare Arzneimittel, nämlich die physiologisch verträgliche, direkt applizierbare pharmazeutische Injektionslösung mit den Komponenten (A) und (B), weist als mengenmäßige Hauptflüssigkeitskomponenten Wasser und Alkohol auf und sein bzw. ihr pH-Wert liegt im neutralen bis schwach sauren Bereich, vorzugsweise im Bereich von pH 6 bis pH 8, besonders bevorzugt im Bereich von pH 6,5 bis pH 7,5.

Gegenstand vorliegender Erfindung ist ferner auch ein Verfahren zur Herstellung einer sterilen injizierbaren flavonhaltigen Pflanzenextrakt-Lösung und eines injizierbaren, flavonhaltige Pflanzenextrakte enthaltenden Arzneimittels. Das Verfahren zur Herstellung einer erfindungsgemäßen sterilen injizierbaren flavonhaltigen Pflanzenextrakt-Lösung ist dadurch gekennzeichnet, daß ein auf herkömmliche Weise gewonnener Pflanzenextrakt aus Gingko biloba und/oder Aesculus spec., vorzugsweise aus deren Blätter und/oder Blüten, in wäßrig-alkoholischem Medium aus 2-40% m/m, bevorzugt 10 - 25 % m/m Alkohol (vorzugsweise Ethanol) in Wasser gelöst wird, daß die dabei erhaltene Lösung auf einen sauren pH-Wert im Bereich von pH 2 bis pH 6, vorzugsweise von pH 3 bis pH 4 eingestellt wird, und daß diese saure Lösung durch Dampf- und/oder Hitzesterilisierung keimfrei gemacht wird.
Das Verfahren zur Herstellung eines injizierbaren, flavonhaltige Pflanzenextrakte enthaltenden Arzneimittels gemäß vorliegender Erfindung ist dadurch gekennzeichnet, daß für die Komponente (A) ein auf herkömmliche Weise gewonnener Pflanzenextrakt aus Gingko biloba und/oder Aesculus spec., vorzugsweise aus deren Blätter und/oder Blüten, in wäßrig-alkoholischem Medium aus 2 - 40 % m/m , bevorzugt 10 - 25 % m/m Alkohol (vorzugsweise Ethanol) in Wasser gelöst wird, daß die dabei erhaltene Lösung auf einen sauren pH-Wert im Bereich von pH 2 bis pH 6, vorzugsweise von pH 3 bis pH 4 eingestellt wird, und daß diese saure Lösung durch Hitzesterilisierung, insbesondere unter Standardbedingungen, vorzugsweise bei 121°C, 2 bar und für die Dauer von 15 Minuten, keimfrei gemacht wird, daß für die Komponente (B) eine Pufferlösung mit einer physiologisch-verträglichen, d.h einen geeigneten pH-Wert und eine geeignete Osmolarität aufweisenden, Stoffzusammensetzung bereitgestellt wird, daß die beiden Komponenten (A) und (B) für die Dauer zwischen Herstellung und Applikation/Injektion/Injizierung in separaten Behältnissen, beispielsweise in zwei Ampullen oder in zwei Kammern einer Mehrkammerspritze oder eines anderen Mehrkammersystems, abgefüllt gelagert werden, und daß sie für den beabsichtigten (in Kürze anstehenden) Injektionseinsatz miteinander vermischt werden.

Die Einstellung des pH-Werts der Komponente (A) erfolgt vorzugsweise durch Zugabe einer Säure, insbesondere Salzsäure.

Bei der Komponente (B) sind als Pufferlösung erfindungsgemäß alle Pufferlösungen geeignet, die üblicherweise in bzw. zur Herstellung von Arzneimitteln verwendet werden.
Besonders bevorzugt ist eine Phosphorsäure/Natriumphosphat-Lösung.

Die Pufferlösung kann wenigstens ein Isotonisierungsmittel, insbesondere Kochsalz und/oder Zuckeralkohol(e) enthalten.

Im folgenden wird die Erfindung anhand von Ausführungsbeispielen näher erläutert.

### Beispiel 1: Herstellung einer Gingko Extrakt enthaltenden Lösung mit dem erfindungsgemäßen Verfahren

| Zutaten: | Menge [% m/m]: |
|---|---|
| Ginkgo Extrakt | 0,916 |
| Sorbit | 4,985 |
| Ethanol 96 % | 19,940 |
| Salzsäure 1 N | 0,474 |
| Wasser für Injektionszwecke | 73,685 |

### Herstellung:

Sorbit wird in 90 % des gesamten Wassers gelöst und der Ethanol zugefügt. Der Extrakt wird zugegeben und unter Rühren gelöst. Durch Zugabe von Salzsäure wird ein pH-Wert von 3,0 eingestellt, anschließend wird mit dem restlichen Wasser auf 100 % Masse aufgefüllt. Diese Lösung wird in der für Injektionszubereitungen üblichen Weise über einen 0,22 µm Membranfilter filtriert, unter Begasung mit sterilem Stickstoff in Ampullen abgefüllt und unter Standardbedingungen im Endbehältnis (15 min, 121 °C; Europäisches Arzneibuch 1997) dampfsterilisiert.

Die abgekühlte Lösung ist klar und frei von Ausfällungen. Eine Bestimmung des Flavonglykosid- und Terpenlactongehalts der Extraktlösung in den Ampullen vor und nach der Dampf- bzw. Hitzesterilisierung zeigt im Vergleich, daß sich der Gehalt an Flavonglykosiden nur um 2,0 % und der Gehalt an Terpenlactonen nur um 3,8 % verringert hat.

Werden 2 ml dieser sterilisierten Extraktlösung mit 2 ml einer geeigneten Pufferlösung gemischt, stellt sich ein physiologisch akzeptabler pH-Wert von 6,8 ein.

### Beispiel 2: Herstellung einer Gingko Extrakt enthaltenden Lösung mit dem erfindungsgemäßen Verfahren

| Zutaten: | Menge [% m/m]: |
|---|---|
| Ginkgo Extrakt | 0,916 |
| Sorbit | 4,985 |
| Propylenglykol 100 % | 19,940 |
| Salzsäure 1 N | 0,474 |
| Wasser für Injektionszwecke | 73,685 |

### Herstellung:

Wie in Beispiel 1 beschrieben, mit der Ausnahme, daß anstelle von Ethanol Propylenglykol zum Einsatz kommt.

### Beispiel 3: Herstellung einer Aesculus spec. Extrakt enthaltenden Lösung mit dem erfindungsgemäßen Verfahren

| Zutaten: | Menge [% m/m]: |
|---|---|
| Aesculus Extrakt | 0,916 |
| Sorbit | 4,985 |
| Ethanol 96 % | 19,940 |
| Salzsäure 1 N | 0,474 |
| Wasser für Injektionszwecke | 73,685 |

### Herstellung:

Wie in Beispiel 1 beschrieben, mit der Ausnahme, daß anstelle von Ginkgo Extrakt Aesculus Extrakt zum Einsatz kommt.

### Beispiel 4: Vergleichsbeispiel

### Zutaten und Mengen [% m/m]:

wie bei Beispiel 1, jedoch unter Verwendung von lediglich 0,2-0,4% Salzsäure.

### Herstellung:

Wie in Beispiel 1 beschrieben, mit der Ausnahme, daß anstelle von pH 3,0 ein pH-Wert von 4,0 eingestellt wird.

Die Bestimmung des Flavonglykosid- und Terpenlactongehalts der Extraktlösung in den Ampullen vor und nach der Dampf- bzw. Hitzesterilisierung zeigt im Vergleich, daß sich der Gehalt an Terpenlactonen durch die Hitzesterilisation um 21,3 % (Abnahme an Bilobalid sogar um 45,2 %) verringert hat.

Wird ein noch höherer pH-Wert eingestellt, nimmt der Gehalt an Terpenlactonen durch die Hitzesterilisation noch stärker ab, und es kann letztendlich zu einem vollständigen Abbau der Terpenlactone kommen

### Beispiel 5: Vergleichsbeispiel

| Zutaten: | Menge [% m/m]: |
|---|---|
| Ginkgo Extrakt | 0,916 |
| Sorbit | 4,985 |
| Ethanol 96 % | 9,940 |
| Salzsäure 1 N | 0,474 |
| Wasser für Injektionszwecke | 83,685 |

### Herstellung:

Wie in Beispiel 1 beschrieben.

Ein Vergleich der Extraktlösung in den Ampullen vor und nach der Dampf- bzw. Hitzesterilisierung zeigt, daß die Hitzesterilisation zu einer signifikanten Zunahme der Trübung der Extraktlösung führt, d.h. zur Ausfällung von Extraktbestandteilen, die sich beim Abkühlen nicht wieder lösen.

## Patentansprüche

1. Injizierbare, flavonhaltige Pflanzenextrakt-Lösung aus Gingko biloba und/oder Aesculus spec., vorzugsweise aus deren Blätter und/oder Blüten, **dadurch gekennzeichnet,**
- **daß** die flavonhaltigen Pflanzenextrakte in einem wäßrig-alkoholischen Lösungsmittel mit einem Gehalt von 2 - 40 % m/m, bevorzugt 10 - 25 % m/m Alkohol in Wasser gelöst sind (vorliegen),
- **daß** der pH-Wert der Lösung im sauren Bereich von pH 2 bis pH 6, vorzugsweise von pH 3 bis pH 4 liegt,
- **daß** die Lösung dampfsterilisiert ist, wobei nach 15 Minuten Dampfsterilisation bei 121°C und 2 bar der Gehalt an Flavonen und Terpenen um maximal 10%m/m verringert ist.

2. Pflanzenextrakt-Lösung nach Anspruch 1, **dadurch gekennzeichnet,**
**daß** als Alkohol Ethanol und/oder Propylenglykol eingesetzt ist.

3. Pflanzenextrakt-Lösung nach Anspruch 1 oder 2, **dadurch gekennzeichnet,**
**daß** die Lösung zur Einstellung des pH-Wertes eine pharmazeutisch akzeptable Säure, insbesondere Salzsäure enthält.

4. Pflanzenextrakt-Lösung nach Anspruch 1 bis 3, **dadurch gekennzeichnet, daß** nach 12 Monaten Lagerung bei 25°C und 60% Luftfeuchtigkeit der
Gehalt an Flavonen und Terpenen um maximal 10%m/m verringert ist.

5. Flavonhaltige Extraktlösungen aus Pflanzenteilen enthaltendes, injizierbares Arzneimittel mit hoher Lagerungs- und Hitzestabilität bezüglich seines Gehalts an pharmazeutisch relevanten Wirkstoffen, **dadurch gekennzeichnet**
- **daß** es aus den beiden Komponenten (A), nämlich einer Pflanzenextrakt-Lösung nach einem der Ansprüche 1 bis 4, und (B), nämlich einer sterilen Puffersalzlösung mit physiologisch-verträglicher Stoffzusammensetzung, besteht, die für die Applikation miteinander vermischt sind,
- **daß** diese beiden Komponenten (A) und (B) separat hergestellt und bis zur Applikation voneinander getrennt in separaten Behältnissen lagerbar sind,
- und **daß** der Gehalt an Flavonen und Terpenen der Komponente A nach 12 Monaten Lagerung bei 25°C und 60% Luftfeuchtigkeit um maximal 10%m/m verringert ist.

6. Arzneimittel nach Anspruch 5, **dadurch gekennzeichnet,**
**daß** die Pufferlösung eine Phosphorsäure/Natriumphosphat-Pufferlösung ist.

7. Arzneimittel nach einem der Ansprüche 5 oder 6, **dadurch gekennzeichnet, daß** die Pufferlösung wenigstens ein Isotonisierungsmittel, insbesondere Kochsalz und/oder Zuckeralkohol(e) enthält.

8. Arzneimittel nach einem der Ansprüche 5 bis 7, **dadurch gekennzeichnet, daß** die Komponente(n) (A) und/oder (B) Ascorbinsäure und/oder wenigstens einen anderen pharmazeutischen Hilfsstoff enthalten.

9. Verfahren zur Herstellung einer sterilen injizierbaren flavonhaltigen Pflanzenextrakt-Lösung nach Anspruch 1, **dadurch gekennzeichnet,**
- **daß** ein auf herkömmliche Weise gewonnener Pflanzenextrakt aus Ginkgo biloba und/oder Aesculus spec., vorzugsweise aus deren Blätter und/oder Blüten, in wäßrig-alkoholischem Medium aus 2 - 40 % m/m , bevorzugt 10 - 25 % m/m, Alkohol in Wasser gelöst wird,
- **daß** die dabei erhaltene Lösung auf einen sauren pH-Wert im Bereich von pH 2 bis pH 6, vorzugsweise von pH 3 bis pH 4 eingestellt wird,
- und **daß** diese saure Lösung durch Hitzesterilisierung, nämlich 15 Minuten Dampfsterilisation bei 121°C und 2 bar keimfrei gemacht wird.

10. Verfahren zur Herstellung eines injizierbaren, flavonhaltige Pflanzenextrakte enthaltenden Arzneimittels nach Anspruch 5, **dadurch gekennzeichnet,**
- **daß** für die Komponente (A) ein auf herkömmliche Weise gewonnener Pflanzenextrakt in wäßrig-alkoholischem Medium aus 2-40% m/m , bevorzugt 10 - 25 % m/m, Alkohol in Wasser gelöst wird,
- **daß** die dabei erhaltene Lösung auf einen sauren pH-Wert im Bereich von pH 2 bis pH 6, vorzugsweise von pH 3 bis pH 4 eingestellt wird, und daß diese saure Lösung durch Hitzesterilisierung, nämlich 15 Minuten Dampfsterilisation bei 121°C und 2 bar keimfrei gemacht wird,
- **daß** für die Komponente (B) eine Pufferlösung mit einer physiologisch-verträglichen Stoffzusammensetzung, bereitgestellt wird,
- **daß** die beiden Komponenten (A) und (B) für die Dauer zwischen Herstellung und Applikation/Injektion/Injizierung in separaten Gefäßen abgefüllt gelagert werden,
- und **daß** sie für den Injektionseinsatz miteinander vermischt werden.

## Claims

1. An injectable, flavone-containing plant extract solution from Ginkgo biloba and/or Aesculus spec., preferably from their leaves and/or flowers **characterised in**
- **that** the flavone-containing plant extracts are dissolved in an aqueous alcoholic solvent with a content of 2 - 40 % m/m, preferably 10 - 25 % m/m alcohol in water,
- **that** the pH of the solution is in the acidic range of pH 2 to pH 6, preferably pH 3 to pH 4,
- and **that** the solution has been sterilized by steam sterilization, whereby after steam sterilization at 121 °C and 2 bar for the duration of 15 minutes, the content of flavones and terpens is reduced by max. 10% m/m.

2. The plant extract solution according to claim 1, **characterised in that** ethanol and/or propylene glycol are used as the alcohol.

3. The plant extract solution according to claim 1, **characterised in that** the solution for adjustment of the pH contains a pharmaceutically acceptable acid, particularly hydrochloric acid.

4. The plant extract solution according to one of the claims 1 to 3, **characterised in that** the content of flavones and terpens is reduced by max. 10 % m/m after 12 months of storage at 25°C and 60% relative humidity.

5. An injectable drug containing flavone-containing plant extract solutions with high storage and heat stability regarding its content of pharmaceutically relevant ingredients , **characterised in**
- **that** it consists of the two components (A), that is a plant extract solution according to one of the claims 1 to 4, and (B), that is a sterile buffer salt solution with physiologically tolerable composition, which are mixed together for the application,
- **that** these two components (A) and (B) are prepared separately and are storeable separately in individual containers until the application,
- and **that** after 12 months of storage at 25°C and 60% relative humidity the content of flavones and terpens of component A is reduced by max. 10% m/m.

6. The drug according to claim 5, **characterised in that** the buffer solution is a phosphoric acid/sodium phosphate buffer solution.

7. The drug according to claims 5 or 6, **characterised in that** the buffer solution contains at least one isotonising agent, particularly table salt and/or sugar alcohol(s).

8. The drug according to one of the claims 5 to 7, **characterised in that** the component(s) (A) and/or (B) contain ascorbic acid and/or at least one other pharmaceutical adjuvant.

9. A method for preparation of a sterile injectable flavone-containing plant extract solution according to claim 1, **characterised in**
- **that** a plant extract, extracted in a conventional way from Ginkgo biloba and/or Aesculus spec., preferably from their leaves and/or flowers, is dissolved in aqueous alcoholic medium of 2 - 40 % m/m, preferably 10 - 25 % m/m alcohol in water,
- **that** the solution obtained in this way is adjusted to an acidic pH in the range of pH 2 to pH 6, preferably pH 3 to pH 4,
- and **that** this acidic solution is sterilised by heat sterilisation, that is steam sterilisation at 121 °C and 2 bar for the duration of 15 minutes.

10. A method for preparation of an injectable flavone-containing plant extract-containing drug according to claim 5, **characterised in**
- **that** a plant extract, extracted in a conventional way, is dissolved in aqueous alcoholic medium of 2 - 40 % m/m, preferably 10 - 25 % m/m alcohol in water for the component (A),
- **that** the solution obtained in this way is adjusted to an acidic pH in the range of pH 2 to pH 6, preferably pH 3 to pH 4,
- **that** this acidic solution is sterilised by heat sterilisation, that is steam sterilisation at 121 °C and 2 bar for the duration of 15 minutes,
- **that** a buffer solution with physiologically tolerable substance composition is provided for component (B),
- **that** both components (A) and (B) are stored filled in individual containers for the time between preparation and application/injection/injecting,
- and **that** they are mixed together for use as injection.

## Revendications

1. Solution d'extrait de plantes à base de ginkgo biloba et/ou de aesculus spec, de préférence à base de leurs feuilles et/ou de leurs fleurs, ladite solution contenant des flavones et pouvant être injectée, **caractérisée en ce que**
• les extraits de plantes contenant des flavones sont dissous (sont disponibles) dans un solvant d'alcool aqueux ayant une concentration comprise entre 2 et 40 % m/m, de préférence comprise entre 10 et 25 % m/m, d'alcool dans l'eau,
• la valeur du pH de la solution se situe dans le domaine acide compris entre pH 2 et pH 6, de préférence entre pH 3 et pH 4,
• la solution est distillée par entraînement à la vapeur, sachant qu'après 15 minutes de distillation par entraînement à la vapeur à 121 °C et 2 bars, la teneur en flavones et en terpènes a diminué d'au plus 10 % m/m.

2. Solution d'extrait de plantes selon la revendication 1, **caractérisée en ce que** de l'éthanol et/ou du propylène glycol sont employés comme alcool.

3. Solution d'extrait de plantes selon la revendication 1 ou 2, **caractérisée en ce que** la solution contient un acide acceptables pharmaceutiquement pour ajuster la valeur du pH, notamment de l'acide chlorhydrique.

4. Solution d'extrait de plantes selon l'une des revendications 1 à 3,
**caractérisée en ce qu'**après 12 mois de stockage à 25 °C et 60 % d'humidité de l'air, la teneur en flavones et en terpènes a diminué d'au plus 10 % m/m.

5. Médicament injectable contenant des solutions d'extrait contenant des flavones à base de parties de plantes, présentant une grande stabilité au stockage et à la température en ce qui conceme la teneur en substances actives ayant une action pharmaceutique, **caractérisé en ce que**
• il se compose de deux composants (A), à savoir une solution d'extrait de plantes selon l'une des revendications 1 à 4, et (B) à savoir une solution stérile de sels tampons ayant une composition compatible physiologiquement, les deux composants étant mélangés ensemble pour l'application ;
• les deux composants (A) et (B) sont fabriqués séparément et peuvent être stockés séparément l'un de l'autre dans des récipients distincts jusqu'à l'application ;
• et la teneur en flavones et en terpènes du composant (A) après 12 mois de stockage à 25 °C et 60 % d'humidité de l'air a diminué d'au plus 10 % m/m.

6. Médicament selon la revendication 5, **caractérisé en ce que** la solution tampon est une solution tampon d'acide phosphorique / phosphate de sodium.

7. Médicament selon la revendication 5 ou 6, **caractérisé en ce que** la solution tampon contient au moins un agent d'isotonisation, notamment du chlorure de sodium et/ou un/des sucre(s) alcool(s).

8. Médicament selon l'une des revendications 5 à 7, **caractérisé en ce que** le composant (A) et/ou le composant (B) contiennent de l'acide ascorbique et/ou au moins un autre adjuvant pharmaceutique.

9. Procédé pour la fabrication d'une solution d'extrait de plantes stérile, injectable et contenant des flavones, selon la revendication 1, **caractérisé en ce que**
• un extrait de plantes obtenu de façon courante à base de ginkgo biloba et/ou de aesculuc spec, de préférence à base de leurs feuilles et/ou de leurs fleurs, est solubilisé dans un milieu alcoolisé aqueux se composant de 2 à 40 % m/m, de préférence de 10 à 25 % m/m d'alcool dans l'eau,
• le pH de la solution ainsi obtenue est ajusté à une valeur acide située dans le domaine compris entre pH 2 et pH 6, de préférence entre pH 3 et pH 4,
• la solution acide est libérée de ses germes par stérilisation à chaud, à savoir par distillation par entraînement à la vapeur à 121 °C et 2 bars pendant 15 minutes.

10. Procédé de fabrication d'un médicament injectable contenant des extraits de plantes contenant des flavones selon la revendication 5, **caractérisé en ce que**
• pour le composant (A) on dissout un extrait de plantes obtenu de façon habituelle dans un milieu alcoolique aqueux se composant de 2 à 40 % m/m, de préférence de 10 à 25 % m/m, d'alcool dans l'eau,
• le pH de la solution ainsi obtenue est ajusté à une valeur acide située dans le domaine compris entre pH 2 et pH 6, de préférence entre pH 3 et pH 4, et cette solution est libérée de ses germes par stérilisation à chaud, à savoir par distillation par entraînement à la vapeur à 121 °C et 2 bars pendant 15 minutes,
• pour le composant (B) on prépare une solution tampon ayant une composition compatibles physiologiquement,
• les deux composants (A) et (B) sont stockés remplis dans des récipients distincts pour la durée séparant la fabrication et l'application / l'injection,
• et ils sont mélangés ensemble pour l'injection.
